# EUROPEAN PATENT APPLICATION

(11) **EP 0 997 146 A1**
(43) Date of publication of application: **03.05.2000**
(21) Application number: 98941215.0
(22) Date of filing: 01.09.1998
(51) Int. Cl.: A61K 31/12

(54) **A DRUG FOR TREATING DIABETIC NEPHROSIS AND DELAYING RENAL FAILURE**

(30) Priority: 12.12.1997 CN 97107229
(71) Applicant: Nanjing General Hospital of Nanjing Command PLA, Nanjing City, Jiang Su 210002 (CN)
(72) Inventor: LI, Leishi, Nanjing City, Jiang Su Province 210002 (CN)
(74) Representative: Stalla-Bourdillon, Bernard
(86) International application number: CN9800176
(87) International publication number: WO9930698

(57) **Abstract**

A drug for treating diabetic nephrosis and delaying renal failure contains emodin. It may be prepared the emodin-containing oral capsule or aqueous solution. The dosage is 100-200 mg/day, such drug can prevent the hypertrophy of kidney of rat with diabetes, and correct an abnormal hemodynamics; can retard the injury of kidney of rat with diabetic nephrosis and protect the function of kidney; can correct the abnormal metabolism of lipoidemia of patient with diabetes; can reduce the growth of extracellular stroma in the renal tissue of diabetic patient, and delay the sclerosis of glomeruli.

## Description

### TECHNICAL FIELD

The present invention relates to anthraquinones, especially to a use of emodin, more particularly an application in preparing medicine.

### BACKGROUND OF THE INVENTION

Diabetes mellitus is a metabolic disease characterized by total or relative insulin deficiency. As an important biological hormone, insulin can regulate the level of glucose, and synthesis of proteins and lipids. Because of insulin deficiency, the structural and functional damages occur in those major organs and tissues such as cardiac, renal, peripheral nervous tissue, and retina in diabetic patients, together with a series of metabolic disorders. Diabetic nephropathy is a frequently occurring long-term complication and a major cause of chronic renal failure (Uremia).

Uuntil now except insulin there has been lack of effective medicines for treatment of diabetes, especially for diabetic nephropathy.

### SUMMARY OF THE INVENTION

The present invention relates to a new application of emodin in preparing medicine for treatment of diabetic nephropathy and/or delaying progression of chronic renal failure.

The object of the present invention is to provide a new use of emodin, that is the new application in preparing medicine, especially the new application in preparing medicine for treatment of diabetic nephropathy and/or delaying progression of chronic renal failure.

The present invention provides a medicine for treatment of diabetic nephropathy effectively, and then delaying progression of renal failure.

The medicine of the present invention contains emodin. The general formula of emodin can be shown as the following:

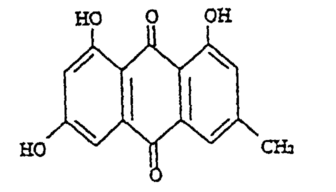

The preparation of the medicine containing emodin provided by the present invention for treatment of diabetic nephropathy and/or delaying progression of chronic renal failure is very simple, in which the emodin may be grounded into powder and then prepared into capsula or solution such as aqueous solution for oral. The dosage of emodin may be in the range of 100 to 200mg per day. The medicine may be also prepared by other common method.

The medicine of the present invention for delaying progression of chronic renal failure can be prepared into complex containing emodin. The medicine of the present invention for delaying progression of chronic renal failure can be prepared into complex containing emodin and captopril, which can be also prepared into capsula or solution such as aqueous solution for oral. The dosage may be 100∼200mg per day for emodin and 50∼150mg per day for captopril. The mixture of medicine can be prepared according to the proportion of daily dosage of emodin and captopril.

### DETAILED DESCRIPTION OF THE INVERTION

The present invention relates to a new application of emodin in preparing medicine for treatment of diabetic nephropathy and/or delaying progression of chronic renal failure.

The object of the present invention is to provide a new use of emodin, that is the new application in preparing medicine, especially the new application in preparing medicine for treatment of diabetic nephropathy and/or delaying progression of chronic renal failure.

The present invention provides a medicine for treatment of diabetic nephropathy effectively, and then delaying progression of renal failure.

The medicine of the present invention contains emodin. The general formula of emodin can be shown as the following:

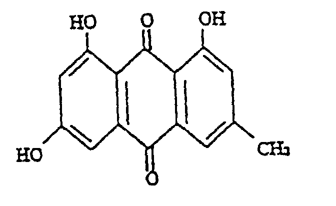

The medicine of the present invention can be prepared into capsula or solution such as aqueous solution for oral. The dosage of emodin may be 100∼200mg per day, and may be given twice or thrice a day.

The present invention provides a medicine for delaying progression of chronic renal failure which contains emodin, and may be prepared into capsula or solution such as aqueous solution containing emodin for oral. The dosage of emodin may be 100 ∼ 200mg per day.

The medicine provided by the present invention for delaying progression of chronic renal failure can also be prepared into complex containing emadin, such as a mixture of emodin and captopril, which may be also prepared into capsula or solution such as aqueous solution for oral. The dosage may be 100∼200mg per day for emodin and 50∼150mg pre day for captopril. The mixture of the medicine can be prepared according to the proportion of daily dosage of emodin and captopril. The medicine may also be prepared by common methods.

Rhubarb (Rheum Officinale) is one of the important herbs in China, mainly produced in Qinghai, Gansu, Ningxia, and Inner Mongolia. Emodin is a major component of anthraquinone derivates extracted from rhubarb. In Rheum palmatum L., the total content of anthraqiunone derivates is 3.5 percent, in which the content of emodin is about 1.27 percent. It has been proved that emodin possesses effects of anti-bacterial, anti-inflammation, and anti-tumor, etc. It has been known that the blood concentration of emodin reached the level of 50 ng/ml in 30 minutes and a peak level of 350∼500 ng/ml in four hours after one single dose of emodin at dosage of 10 mg/kg in female SD rats (body weight: 200±30g). The blood level of emodin decreases below 50 ng/ml at 12th hour in female SD rats. Emodin is of very low toxicity. None of rats died after one single dose of emodin at dosage of 3.0g/Kg in adult male or female SD rats. Our experimental data proved that not only emodin can be used as the medicine for treatment of diabetic nephropathy, but it also delays the progression of chronic renal failure.

The increase of triglyceride and cholesterol in diabetic rats can be controlled by emodin given orally. By the isolated perfused kidney technique (IPK), the metabolic level of kidney tissues from diabetic rats treated by emodin can be controlled significantly, which showed as the decrease of oxygen consumption and clearance of inulin. The kidney weight and the contents of protein, DNA, and RNA in kidney tissues were decreased in emodin treated group in comparison to those in control group.

In hemodynamic study of kidneys from diabetic rats, the clearance of inulin and renal blood flow decreased by emodin given, and the filtration fraction appeared decreasing tendency.

By morphological study of kidney tissues from diabetic rats, the volume of glomeruli and the area of mesangium reduced obviously by emodin administered. Emodin could suppress the production of fibronectin (FN) from mesangial cells, and decrease the hillock numbers of mesangial cells.

In brief, emodin possess effectiveness as follows:
1. Emodin can prevent renal hypertrophy of diabetic nephropathy rats, and ameliorate its abnormally hemodynamic changes;
2. Emodin can reduce the damage of kidney in diabetic nephropathy rats, and protect the kidney function;
3. Emodin can correct the abnormally metabolism of lipid in diabetes;
4. Emodin can decrease the production of extracellular matrix in kidney tissues from diabetic rats, and ameliorate the progression of glomerularsclerosis.

As shown in further investigation, emodin possesses the effect on delaying the progression of chronic renal failure in rat. Emodin administered orally is able to suppress the kidney index, reduce the content of protein, DNA and RNA in kidney tissues, decrease the levels of serum creatinine and urea nitrogen, normalize the levels of plasma lipid, ameliorate the urinary protein excretion, reduce the oxygen consumption and re-production glucose of kidney and suppress the growth of mesangial cells and proliferation of tubular epithelia cells. Thus, emodin can delay progression of chronic renal failure.

The present invention will be illustrated in more details in the following examples.

### BRIEF DESCRIPTION OF THE DRAWINGS

- Figure 1: shows an effect of emodin on the urinary protein exaction in diabetic rats.
- Figure 2a: shows a comparison about the mean volume of glomeruli in two groups from different time.
- Figure 2b: shows a comparison about the mean area of mesangium in two groups from different time.
- Figure 3: shows an effect of emodin on the changes of fibronenctin level in supernatant of cultured mesangial cells.
- Figure 4: shows an effect of emodin on the changes fibronenctin adhered to mesangial cells.
- Figure 5: shows an effect of emodin on the numbers of hillock established by mesangial cells.
- Figure 6: shows a suppression of proliferation of mesangial cells by the sera obtained from rats treated by emodin at various periods.
- Figure 7: shows an effect of various dosages of emodin on the proliferation of rat mesangial cells;
- Figure 8 A: shows an effect of various concentrations of emodin on the proliferation of rat mesangial cells.
- Figure 8 B: shows an effect of various concentrations of emodin on the proliferation of human mesangial cells.
- Figure 9: shows a suppression of proliferation of tubular epithelial cells by the sera obtained from rat treated by various dosages of emodin.
- Figure 10: shows a suppression of proliferation of tubular epithelial cells by the sera obtained from rats treated by emodin at one single dosage or for one week.
- Figure 11: shows an effect of pure emodin on the proliferation of tubular epithelial cells.

### EXAMPLE 1

### Therapy effect of emodin on diabetic nephropathy

### 1. Effect of emodin on the experimental diabetic nephropathy rats

### 1.1. Establishment of diabetic nephropathy animal model of rat

The diabetic nephropathy animal model of rat was established by unilateral nephrotomy and tail vein injection of Streptozotocin (STZ, Sigma Chemical Co.) according to method described briefly as the follows. Adult female SD rats with body weights of 180±20 grams were unilateral nephrotomized under the abdominal anesthesia and aseptic condition. Seven days later after operation, rats were injected by STZ (35mg/Kg) through the tail vein under mild ether anesthesia. Blood glucose level was measured at 72 hours after injection of STZ. Only rats with blood glucose level above 250 mg/ml were recognized as the establishment of diabetic nephropathy and enrolled into the study.

### 1.2. Experimental design and grouping

Experimental rats were divided into four groups: uninephrotomized control group (n=6), diabetic control group (n=18), diabetic emodin-treated group (n=10) and normal control group (n=9). Rats in diabetic emodin-treated group were given aqueous solution of emodin (emodin concentration: 2 mg/ml) orally at a dose of 10 mg/Kg/day in 1 ml/200g (body weight). Rats in control groups were received same quantity of distilled water. After 120 days of treatment, all rats were killed for observation of the effects of emodin therapy.

### 1.3. Results

### 1.3.1. Changes of the general status, such as blood glucose level and body weight.

The body weights of rats from four groups were no markedly difference before experiment, and slightly decreased after uninephrotomy, but the degrees of reduce were not more than 10 percent. The body weights of diabetic rats were reduced obviously after the injection of STZ as compared with that of rats in normal control group. There was no remarkable difference about the body weights between diabetic rats treated with and without emodin.

As shown in the results, the blood glucose levels of rats from four groups were not different before injection of STZ. The blood glucose levels of rats were elevated obviously after injection of STZ, and were different significantly from normal control rats (17.63±1.17 mmol/L vs 6.32±0.34 mmol/L; P<0.01). That means that the establishment of diabetic rats succeeded. No remarkable difference was found in blood glucose levels between the rats from two diabetic groups treated with or without emodin (22.1 ± 3.1 mmol/L vs 20.4 ± 2.6 mmol/L; P>0.05). But the glucose level of diabetic rats was higher than that of normal control rats, which shows that emodin possesses no markedly effect on the hyperglycemia.

### 1.3.2. Effect of emodin on the metabolism of lipid in diabetic rats

The plasma lipid levels of rats from four groups were not different before the experiment. Abnormal metabolism of lipid occurred in diabetic rats after injection of STZ, that is the increment of triglyceride and cholesterol. After treatment of emodin, the increase of triglyceride and cholesterol in diabetic rats were controlled obviously (as shown in table 1), that is the lessening of plasma triglyceride and cholesterol level, the increment of plasma high-density lipoprotein (HDL), and the decline of plasma very low-density lipoprotein (LDL) and very low-density lipoprotein (VLDL).

### 1.3.3. Effect of emodin on metabolic level of kidney tissue in diabetic rats

The metabolic level of kidney tissue can be measured by IPK technique. As shown in table 2, the clearance of inulin (Cᵢₙ) and the volume of urine flow (UV) increased markedly, and oxygen consumption (QO₂) enhanced significantly in diabetic rats compared with that in normal control rats. After treatment of emodin, the metabolic level of kidney tissue was controlled effectively in diabetic emodin-treated rats, which showed as the diminishment of oxygen consumption of kidney compared with that in diabetic control rats. Meanwhile, the clearance of inulin was lower in diabetic emodin-treated rats than that in diabetic control rats.

**Table 1**

| Effect of plasma lipid level and metabolism of lipoprotein in diabetic rat (X±s) | | | |
|---|---|---|---|
| | Group A n = 9 | Group B n = 10 | Group C n = 10 |
| Triglyceride, mmol/L | 0.67 ± 0.13 | 1.87 ± 0.52* | 1.53 ± 0.40*▲ |
| Cholesterol, mmol/L | 3.49 ± 0.71 | 5.14 ± 1.09* | 3.91 ± 1.12*▲ |
| HDL-CH, mmol/L | 1.49 ± 0.29 | 1.47 ± 0.27 | 1.86 ± 0.42*▲ |
| LDL-CH, mmol/L | 1.71 ± 0.51 | 2.70 ± 0.69* | 1.38 ± 0.47 ▲ |
| VLDL-CH, mmol/L | 0.32 ± 0.06 | 0.99 ± 0.13* | 0.82 ± 0.19* |

| | | | |
|---|---|---|---|
| Note: *=P<0.01 vs group A; | | | |
| ▲:=P<0.05 vs group B(sameness as the following) | | | |
| Group A=normal control group; Group B=diabetic control group; Group C=diabetic emodin-treated group | | | |

**Table 2**

| Effect of emodin on whole renal function and oxygen consumption of IPK in diabetic rat (X±s) | | | |
|---|---|---|---|
| | Group A n = 9 | Group B n = 6 | Group C n = 6 |
| MAP, kPa | 16.26 ± 1.22 | 21.06 ± 3.17* | 20.23 ± 3.67*▲ |

| C_{In,} | | | |
|---|---|---|---|
| Ml/mm | 1.04 ± 0.27 | 1.42 ± 0.30** | 1.37 ± 0.21 |
| Ml/min × g⁻¹ | 0.48 ± 0.11 | 0.58 ± 0.18 | 0.67 ± 0.13 |
| UV, ml/min | 0.026 ± 0.014 | 0.082 ± 0.031* | 0.058 ± 0.029* |

| QO₂ | (n=4) | (n=6) | (n=4) |
|---|---|---|---|
| Basal QO_{2.} µmol/min | 2.48 ± 0.37 | 4.37 ± 0.97* | 3.10 ± 0.52*▲ |
| Total QO_{2,} µmol/min | 3.03 ± 0.62 | 4.86 ± 1.06* | 3.79 ± 0.89*▲ |

| | | | |
|---|---|---|---|
| Note: * = P<0.01, | | | |
| ** = P<0.05 vs group A; | | | |
| ▲ = P<0.05 vs group B | | | |
| MAP = mean arterial pressure; Cin = clearance of inulin; UV = volume of urine flow O₂ = Oxygen consumption | | | |

### 1.3.4. Effect of emodin on the renal hypertrophy

Renal hypertrophy is one of the most characteristic morphological changes in diabetic nephropathy. In present study, kidney weight increased significantly, and the content of protein, DNA, and RNA in kidney tissues also elevated obviously in diabetic rats, but the degrees of enhance of protein and RNA were greater than that of DNA. As shown in table 3, the kidney weight and the content of protein, RNA and DNA in kidney tissues from diabetic emodin-treated group were obviously lower than that from diabetic control group.

**Table 3**

| Effect of emodin on kidney weight and the content of protein, RNA and DNA in kidney tissues from diabetic rat (X±s) | | | |
|---|---|---|---|
| | Group A n = 9 | Group B n = 10 | Group C n = 10 |
| KW, g | 2.23 ± 0.17 | 2.94 ± 0.27* | 2.54 ± 0.25* |
| KW/BW, % | 6.85 ± 0.62 | 13.5 ± 0.83* | 10.4 ± 0.47*▲ |

| In kidney tissues mg/g(kidney) | | | |
|---|---|---|---|
| Protein | 183.7 ± 22.0 | 251.0 ± 34.9** | 207.2 ± 34.5*▲ |
| RNA | 4.26 ± 0.29 | 5.82 ± 0.41* | 5.36 ± 0.67* |
| DNA | 3.04 ± 0.42 | 3.51 ± 0.29* | 340 ± 0.23 * |
| RNA/DNA | 1.41 ± 0.63 | 1.76 ± 0.44* | 1.57 ± 0.53* |

| | | | |
|---|---|---|---|
| Note: * = P<0.01, | | | |
| ** = P<0.05 vs group A; | | | |
| *▲ = P<0.05 vs group B; KW = kidney weight; BW = body weight | | | |

### 1.3.5. Effect of emodin on the urinary protein excretion

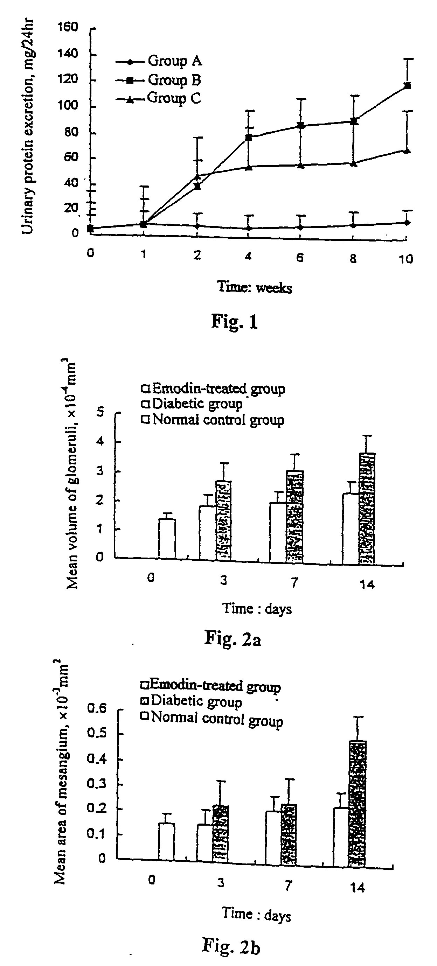

As shown in Figure 1, the 24-hour urinary protein excretion in diabetic rats increased significantly compared with that in normal control rats. After emodin therapy, the 24-hour urinary protein excretion reduced markedly in diabetic emodin-treated group.

### 2. Effect of emodin on the hemodynamic changes of kidney in diabetic rats

### 2.1.Diabetic animal model of rat

Adult female SD rats with body weights of 170 to 190 grams were used. Rats were made diabetes by an injection of STZ in a dose of 40 mg/Kg through tail vein. The blood glucose level was measured at 72th hour after injection of STZ. Only rats with blood glucose level within the range of 15.3 to 25 mmol/L were enrolled into the study.

### 2.2. Experimental design and grouping

Rats were divided into three groups, normal control group (n=6), diabetic group (n=11) and emodin-treated diabetic group (n=9). Rats in diabetic emodin-treated group received treatment of emodin in a dose of 10 mg/Kg orally at 18th hour after injection of STZ, and then the emodin-treated continued for four weeks in a dose of 10 mg/Kg/day orally.

### 2.3. Results

### 2.3.1. Changes of blood pressure

The blood pressures of rats from three groups were within the range of 11.2 to 14.0 kPa, and no markedly difference before the experiment. The mean arterial pressure (MAP) increased obviously in rats received injection of STZ (15.9±0.5 vs 13.8±0.8 kPa, p<0.05). There were no obviously changes of MAP in diabetic rats after treated by emodin.

### 2.3.2. Changes of hemodynamics

As shown in table 4, the clearance of inulin and renal blood flow enhanced significantly, the resistance of renal blood vessels reduced, and filtration fraction (FF) elevated in diabetic rats as compared with the normal control rats. After treatment of emodin, the clearance of inulin and renal blood flow decreased obviously in emodin-treated diabetic rats, and meanwhile FF appeared the tendency of diminishing.

**Table 4**

| Effect of emodin on renal hemodynamics in diabetic rats | | | |
|---|---|---|---|
| | Groups | | |
| | Normal control n = 6 | Diabetic n = 11 | Emodin-treated n = 9 |
| MAP, kPa | 13.8 ± 0.8 | 15.9 ± 0.5 | 16.0 ± 0.9 |
| C_{In}, ml/min | 1.67 ± 0.22 | 3.30 ± 0.94 | 2.15 ± 0.44 |
| RBF, ml/ml | 6.01 ± 0.23 | 8.63 ± 0.48 | 7.14 ± 0.31 |
| RVR, mmHg/min/ml | 7.31 ± 0.28 | 9.06 ± 0.41 | 8.11 ± 0.37 |
| FF | 0.28 ± 0.03 | 0.38 ± 0.07 | 0.33 ± 0.04 |
| UV, ul/min | 7.3 ± 2.1 | 34.7 ± 16.3 | 28.6 ± 14.2 |
| Note: MAP = mean arterial pressure; C_{In} clearance of inulin; RBP = renal blood flow; RVR = renal vessel resistance; FF = filtration fraction; UV volume of urine flow | | | |

### 3. Morphological analysis of glomeruli from diabetic rats treated with emodin

### 3.1. Diabetic animal model of rat was established in the same manner as described above.

### 3.2. Experimental design and grouping

Rats were divided into three groups, normal control group (n=12), diabetic group (n=12) and emodin-treated diabetic group (n=12). Diabetic rats were treated with emodin in the same manner as mentioned above. Four rats chosen randomly from each group were killed at day 3, day 7, and day 14 respectively after treatment of emodin. Kidney tissue from each rat was routinely pathologically examined. Morphological changes of kidney tissues from each group were quantitated by the image analysis technique under microscope.

### 3.3. Results

As shown in Figure 2, the volume of glomeruli enlarged obviously in rats after injection of STZ compared with the normal rats. The volume of glomeruli and area of mesangium diminished significantly in=emodin-treated diabetic rats at day 3, day 7, and day 14 after occurrence of diabetes compared with the diabetic rats without emodin therapy.

### 4. Effect of emodin on the production of fibronectin from cultured mesangial cells

### 4.1. Culture of mesangial cells

Human glomeruli were isolated by machinery sieve method from the normal adult kidney cortex tissues, digested by 0.1% collagenase, maintained in DMEM supplemented with 2mM glutamine and 20% fetal bovine serum. Mesangial cells from glomeruli between passages 3 to 5 were used in the experiment described as following.

### 4.2. Measurement of production of fibronection (FN)

The content of FN in supernatant and FN deposition of cultured mesangial cells was examined by ELASA and immunofluorescence techniques.

### 4.3. Results

### 4.3.1. Effect of emodin on the fibronection production of mesangial cells

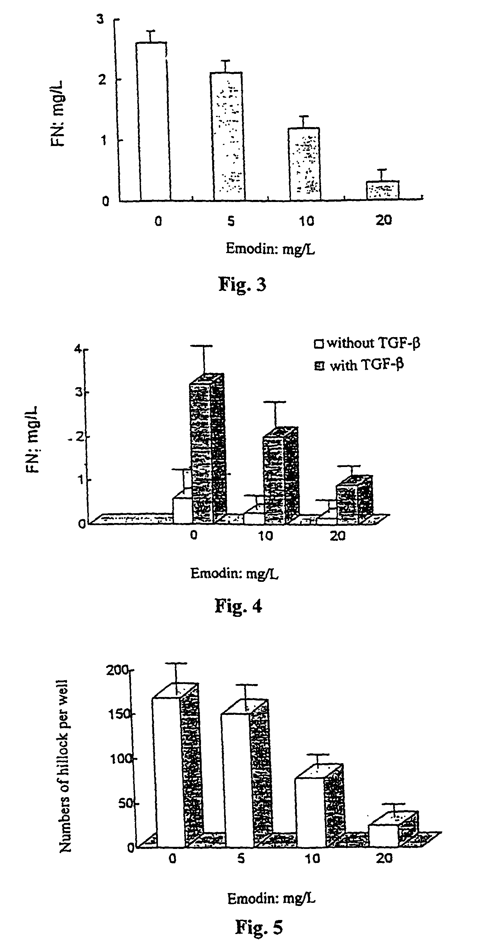

The amount of FN in the supernatants of mesangial cells decreased significantly after exposure to emodin. As shown in Figure 3 and Figure 4, emodin at concentration of 20 mg/L almost abolished the FN production and deposition of mesangial cells with transforming growth factor-β stimulation.

### 4.3.2. Effect of emodin on the hillock formation of mesangial cells

The hillock formation was pile up growth of the long-term cultured mesangial cells due to the proliferation of mesangial cell and accumulation of extracellular matrix. Many large hillocks constructed by mesangial cell cultured without emodin. Emodin does dependently delayed and lessened the formation of hillock, and almost abolished the hillock of mesangial cells when emodin at concentration of 20 mg/L.

The conclusions of Example 1 can be summarized as follows:
1. Emodin can prevent the renal hypertrophy, and normalize the hyper-hemodynamics in diabetic rat.
2. Emodin can ameliorate renal damage, and protect the renal function in diabetic rat.
3. Emodin can correct abnormal lipid metabolism in diabetes mellitus.
4. Emodin can lessen the production of extracellular matrix in kidney tissues, and delay progression of glomerulosclerose in diabetic rat.

### EXAMPLE 2

### Effect of emodin on the progression of chronic renal failure

### 1. Effect of emodin on the progression of chronic renal failure in rat

### 1.1. Animal model of chronic renal failure

Animal model of chronic renal failure was established by 5/6 nephroctomy. Adult male SD rats with body weights of 150 to 200 grams were used. Upper and lower arterial branches of left renal artery were ligated first, right kidney were nephroctomized after seven days ligation of artery under the abdominal anesthesia and aseptic condition, and the time of operation was recorded.

### 1.2. Experimental design and grouping

Rats were divided into three groups after four weeks of operation, normal control group (n=10), emodin-treated 5/6 nephroctomy group (Emodin group in short; n=16) and 5/6 nephroctomized control group (Control group in short; n=17). Rats in emodin group were given aqueous solution of emodin orally at a dose of 10 mg/Kg/day. Rats in control groups were received same quantity of distilled water.

### 1.3. Results

### 1.3.1. Effects of emodin on body weight and kidney weight

The body weights of rats in three groups were not markedly different before experiment, and decreased temporarily after operation. In the process of experiment, the body weights of all rats increased progressively, and there was no difference among groups. Twelve weeks after operation, the kidney weights occurred tendency of reducing and the ratio of kidney weight to body weight was lessened significantly in emodin group compared with that in control group. As shown in table 5, the content of protein, DNA and RNA in kidney tissues also decreased obviously in emodin group.

### 1.3.2. Effect of emodin on the renal function

The levels of serum creatinine (S_{Cr}) and blood urea nitrogen (BUN) in three groups were not different before experiment. After 5/6 nephroctomy, the levels of S_{Cr} and BUN elevated sharply, and returned near to normal value in 2 to 4 weeks later. Then the levels of S_{Cr} and BUN increased progressively again in three groups. However, the elevation of the levels of S_{Cr} and BUN in emodin group were slower obviously than that in control group. As shown in table 6, the difference of BUN between emodin group and control group is significant.

### 1.3.3. Effect of emodin on the metabolism of lipid and lipoprotein

The levels of plasma lipid and lipoprotein were not different between emodin group and control group before experiment. As shown in Table 6, the plasma lipid levels were lower significantly in rats received emodin therapy for 12 weeks than that in the control group.

### 1.3.4. Effect of emodin on the urinary protein excretion

The 24-hour urinary protein excretions were not different among rats in three groups before experiment. After operation, the urinary protein excretion increased markedly in 5/6 nephroctomized rats (28.6±7.3 vs 7.1±4.8 mg/24h, P<0.01), and then in the process of experiment the urinary protein excretion elevated progressively. By treatment of emodin, the urinary protein excretion was lower in emodin group than that in control group. The difference of urinary protein excretion was obvious at 12th week between emodin group and control group (35.6±13.7 vs 120.8±38.2 mg/24h, P<0.01).

**Table 5**

| Changes of body weight, kidney weight, content of protein, DNA and RNA in kidney tissues | | | |
|---|---|---|---|
| | Groups | | |
| | Normal control | Emodin | Control |
| BW , g | 372 ± 34 | 350 ± 31 | 369 ± 38 |
| KW , g | 2.01 ± 0.31 | 2.75 ± 0.63 | 3.03 ± 0.36 |
| KW/BW % | 5.31 ± 0.66 | 6.50 ± 1.03 | 7.61 ± 0.91 |

| In kidney tissues: mg/kidney | | | |
|---|---|---|---|
| Protein | 134.3 ± 9.5 | 156.2 ± 17.1 | 183.8 ± 19.5 |
| RNA | 4.13 ± 0.17 | 4.25 ± 0.21 | 4.87 ± 0.45 |
| DNA | 3.51 ± 0.23 | 3.38 ± 0.30 | 4.18 ± 0.43 |
| RNA/DNA | 1.25 ± 0.37 | 1.15 ± 0.42 | 1.26 ± 0.26 |
| Food taken, g/24h | 28.5 ± 8.2 | 26.7 ± 9.5 | 25.3 ± 8.3 |

**Table 6**

| Effect of emodin on the level of S_{Cr} and BUN, plasma lipid and lipoprotein and serum protein | | | |
|---|---|---|---|
| | Groups | | |
| | Normal control | Emodin | Control |
| S_{Cr}: µmol/L | 68.4 ± 21.3 | 190.0 ± 69.5 | 272.6 ± 56.8 |
| BUN: mmol/L | 17.4 ± 2.0 | 21.9 ± 3.8 | 31.7 ± 8.9 |
| Triglyceride: mmol/L | 0.67 ± 0.13 | 1.51 ± 0.42 | 2.06 ± 0.51 |
| Cholesterol: mmol/L | 2.95 ± 0.61 | 3.26 ± 0.70 | 4.26 ± 0.89 |
| HDL-CH: mmol/L | 1.27 ± 0.25 | 1.44 ± 0.31 | 1.10 ± 0.18 |
| LDL-CH: mmol/L | 1.44 ± 0.43 | 1.18 ± 0.35 | 2.06 ± 0.40 |
| VLDL-CH: mmol/L | 0.27 ± 0.05 | 0.61 ± 0.32 | 0.84 ± 0.35 |
| Total protein: g/L | 75.9 ± 3.2 | 72.8 ± 3.4 | 71.2 ± 8.1 |
| Albumin: g/L | 40.1 ± 5.4 | 37.4 ± 3.8 | 36.9 ± 4.7 |
| A/G | 1.12 ± 0.30 | 1.07 ± 0.41 | 1.03 ± 0.38 |
| Na⁺: mmol/L | 141.7 ± 1.6 | 142.2 ± 1.8 | 143.7 ± 1.7 |
| K⁺: mmol/L | 6.2 ± 0.8 | 6.5 ± 0.6 | 6.4 ± 0.8 |
| Note: A/G = Albumin/Globulin | | | |

### 1.3.5. The rate of the death rate in three groups

As shown in Table 7, many rats died in 4 weeks after operation. In the process of experiment, the number of rats died were more in control group than that in emodin group. At end paint of experiment of 12th week, the survival rates of rats were 90%, 75%, and 61 % in three groups respectively.

**Table 7**

| The number of death rats and the survival rate | | | |
|---|---|---|---|
| | Groups | | |
| | Normal control | Emodin | Control |
| Time after operation | | | |
| 4^{th} week | | 2 | 3 3 |
| 7^{th} week | | | 1 |
| 8^{th} week | | 1 | 1 |
| 12^{th} week | 1 | 1 | 1 |
| 16^{th} week | | | 1 |
| Number of survival | 9 | 12 | 11 |
| Survival rate: % | 90 | 75 | 61 |

### 2. Protection of emodin on remnant kidney function of 5/6 nephroctomized rats

### 2.1. Animal model

Animal model of chronic renal failure was established by 5/6 nephroctomy in the same manner as described above.

### 2.2. Experimental design and grouping were the same as mentioned before.

### 2.3. Results

### 2.3.1. Effect of emodin on the renal function of normal rats

As shown in Table 8, there were no difference in the renal functions, such as oxygen consumption, transportation of sodium, clearance of inulin, and re-production of glucose, whether the rats with or without received emodin therapy.

**Table 8**

| Effect of emodin on renal function of normal rats | | |
|---|---|---|
| | Groups | |
| | Normal n = 24 | Emodin-treated normal n = 4 |
| KW: g | 1.62 ± 0.14 | 1.59 ± 0.10 |
| QO₂: µmol/min/g | 4.49 ± 0.72 | 4.66 ± 0.59 |
| T_{Na}⁺: µmol/min/g | 38.87 ± 4.38 | 36.21 ± 5.67 |
| C_{In}: µl/min/g | 282.5 ± 48.5 | 274.8 ± 27.8 |
| Q: ml/min/g | 21.3 ± 2.8 | 22.1 ± 1.7 |
| GP: µmol/min/g | 10.4 ± 3.1 | 8.2 ± 1.6 |
| UV: µl/min/g | 51 ± 7 | 55 ± 9 |
| Note: KW = kidney weight; QO₂ = oxygen consumption; T_{Na}⁺ = transportation of sodium; C_{In} = clearance of inulin; Q = perfusion flow; GP = re-production of glucose; UV = urine flow | | |

### 2.3.2. Effect of emodin on the renal function of remnant kdiney

As shown in Table 9, the oxygen consumption and re-production of glucose were lower obviously in 5/6 nephroctomized rats received treatment of emodin than that in the control group.

**Table 9**

| Effect of emodin on renal function of remnant kidney | | |
|---|---|---|
| | Groups | |
| | Control n = 6 | Emodin-treated n = 6 |
| KW, g | 1.26 ± 0.11 | 1.23 ± 0.28 |
| QO₂, µmol/min/g | 5.74 ± 0.72 | 4.42 ± 0.64 |
| T_{Na}⁺, µmol/min/g | 10.5 ± 2.1 | 9.3 ± 1.5 |
| C_{In}, µl/min/g | 127.4 ± 23.6 | 122.2 ± 13.7 |
| Q, ml/min/g | 14.7 ± 1.6 | 14.1 ± 2.3 |
| GP, µmol/min/g | 10.6 ± 2.9 | 6.0 ± 2.5 |
| UV, µl/min/g | 20 ± 3 | 17 ± 2 |
| Note: KW = kidney weight; QO₂ = oxygen consumption; T_{Na}⁺ = transportation of sodium; C_{In} = clearance of inulin; Q = perfusion flow; GP = re-production of glucose; UV = urine flow | | |

### 3. Suppression of emodin on the growth of mesangial cells and tubular epithelial cells

### 3.1. Suppression of the sera containing emodin on the growth of mesangial cells

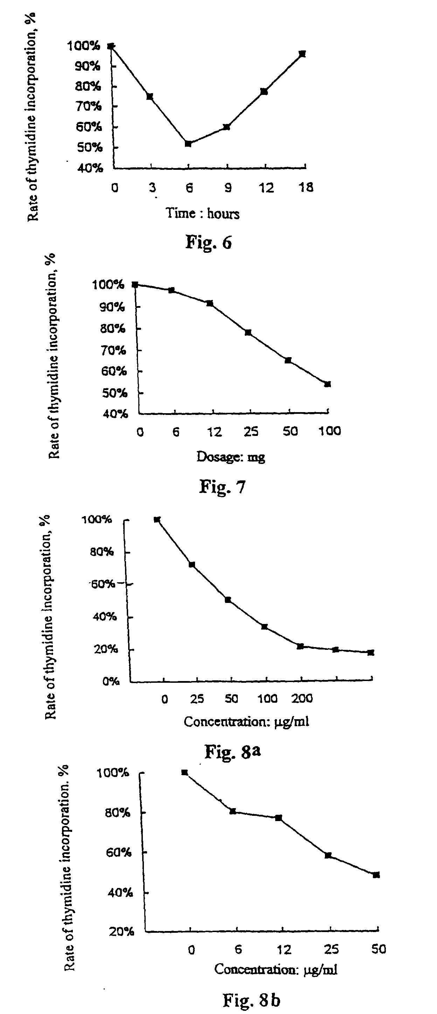

Growth of mesangial cells can be suppressed by the sera from normal rats received one single dose of emodin at 20 mg orally. Suppression of the sera taken at 3th hour on the proliferation of mesangial cells can be observed, and the rate of thymidine incorporation was 77 %. As shown in Figure 6, suppression of the sera taken at 6th hour on the growth of mesnagial cells was most potent, the rate of thymidine incorporation was 60 %, and suppression of the sera taken at 9th hour on the growth of mesangial cells became weakening.

As shown in Figure 7, suppression of the sera on the growth of mesnagial cells was strengthened with the increase of the dosage, in which the sera were taken at the 6^{th} hour after the rats received various dosages of emodin orally

### 3.2. Suppression of emodin on the growth of mesngial cells

As shown in Figure 8, the proliferation of mesangial cells was suppressed by culture medium with emodin. The thymidine incorporation rate of mesangial cells decreased to 58% by emodin at concentration of 25µg/ml. As the concentration of emodin increases, the thymidine incorporation rate of mesangial cells was only 17% at concentration of 100µg/ml.

### 3.3. Effect of emodin on proliferation of tubular epithelial cells

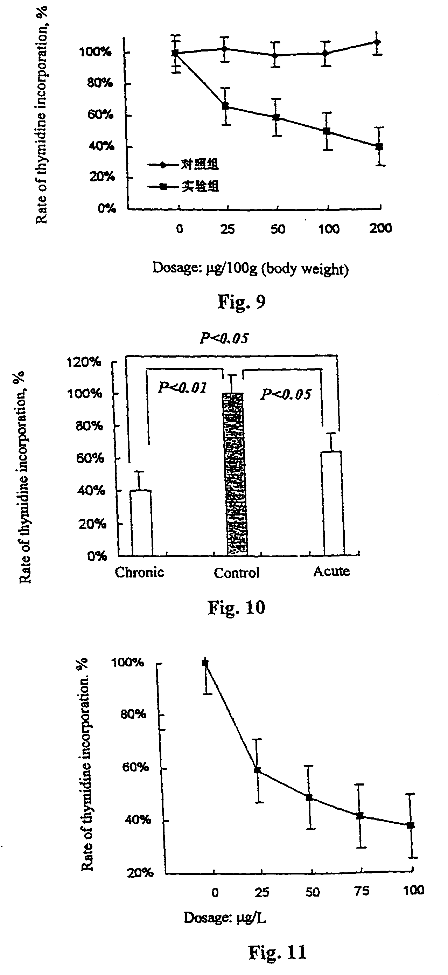

As shown in Figure 9, the sera from normal rats received emodin orally can inhibited the growth of tubular epithelial cells obviously. In Figure 10, suppression of the sera from normal rats given long-term emodin orally on proliferation of tubular epithelial cells was stronger than that received one single dose of emodin. The thymidine incorporation rate of cultured tubular epithelial cells decreased markedly by emodin added to culture medium directly as shown in Figure 11.

### EXAMPLE 3

### Clinical trial of emodin

### 1. Effect of emodin on delaying progression of renal function deteriorating in chronic renal failure patients

### 1.1. Selectivity of patients

Forty-nine diabetes chronic renal failure patients, which male and female cases were 38 and 11 respectively with an average age of 40.7 years, were enrolled in this study. The mean baseline level of serum creatinine was 344±114µmol/L in the patients, after clearance of the reasons, such as disturbance of electrolyte, acid-base abnormal, infections, and hypertension, that caused renal function temporary decreased.

### 1.2. Grouping

Patients were divided into 4 groups randomly, emodin-treated group (n=12), captopril-treated group (n=10), combined treatment group (n=14) and control group (n=13). Patients in emodin-treated group received emodin at dose of 100 to 200 mg per day, patients in captopril-treated group received captopril at dose of 75 mg per day, patients in combined treatment group received 100 to 200 mg of emodin and 75 mg of captopril at the same time per day, and patients in control group only received vehicle as control.

### 1.3. Results

In 6 to 20 months of the follow-up period, 5 patients in control group progressed into end-stage renal failure and had to receive dialysis therapy. None of patient in the other three groups entered into end-stage renal failure. As shown in table 10, the deteriorating of renal function was fastest in control group, next in captopril-treated group, and then in emodn-treated group according to regression analysis.

**Table 10**

| Effect of emodin on delay progression of renal function deteriorating in chronic renal failure patients | | | | |
|---|---|---|---|---|
| | Control | Emodin-treated | Captopril-treated | Combined treatment |
| Patient, No. | 19 | 11 | 9 | 10 |
| Time^{a}, months | 11.8±3.3 | 11.4±4.8 | 9.3±2.8 | 11.7±4.7 |
| RFI^{b}, ×10⁻⁴ | -100.5±46.9 | -6.1±2.1 | -31.9±3.1 | 16.7±4.3 |
| ESRF^{c} | 94.7% | 36.4% | 66.4% | 30% |

| | | | | |
|---|---|---|---|---|
| Note: a = follow-up time; | | | | |
| b = Index of renal failure; | | | | |
| c = presumption that occurrence of end-stage renal failure in 10 years | | | | |

## Claims

1. A medicine for treatment of diabetic nephropathy and/or delaying the progression of chronic renal failure, wherein it contains emodin with the following general formula:

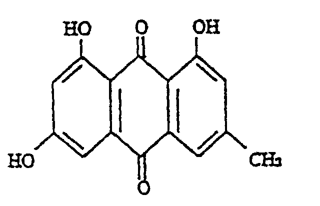

2. A medicine compound for delaying the progression of chronic renal failure, wherein it contains emodin and captopril, the general formaled of emedin is as the following:

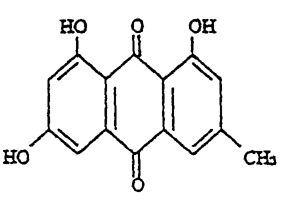

3. A use of the medicine in accordance with claim 1 in treating diabetic nephropathy and/or delaying the progression of chronic renal failure, wherein the dosage of emodin is 100-200mg per day.

4. A use of the medicine compound in accordance with claim 2 in delaying the progression of chronic renal failure, wherein the dosages of emodin and captopril are 100-200mg per day and 50-150mg per day respetively.

5. The medicine compound in accordance with claim 2, wherein it is supplied in capsula or aqueous solution for oral.

6. An application of emodin in preparing the medicine for treatment of diabetic nephropathy and/or delaying the progression of chronic renal failure, wherein the said emodin is shown in the following general formula:

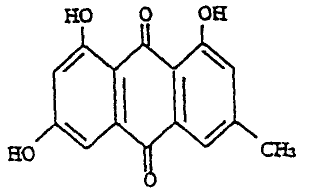

7. The application in accordance with claim 6, wherein the dosage of emodin is 100-200mg per day.

8. The application in accordance with claim 6 and claim 7, wherein the said medicine is supplied in capsula or aqueous solution.
